**Europäisches Patentamt**

**European Patent Office**

**Office européen des brevets**

(11) Numéro de publication: **0 214 887**
**B1**

(12) **FASCICULE DE BREVET EUROPEEN**

(45) Date de publication du fascicule du brevet:
**04.10.89**

(21) Numéro de dépôt: **86401728.0**

(22) Date de dépôt: **01.08.86**

(51) Int. Cl.⁴: **H05G 1/30**, H05G 1/46

(54) **Procédé de réglage d'un dispositif de radiologie.**

(30) Priorité: **02.08.85 FR 8511887**

(43) Date de publication de la demande:
**18.03.87 Bulletin 87/12**

(45) Mention de la délivrance du brevet:
**04.10.89 Bulletin 89/40**

(84) Etats contractants désignés:
**BE DE LU NL**

(56) Documents cités:
**EP-A- 0 022 295**
**EP-A- 0 063 644**
**DE-A- 3 324 537**
**FR-A- 2 395 670**
**FR-A- 2 418 948**
**FR-A- 2 445 088**
**US-A- 4 439 867**

(73) Titulaire: **GENERAL ELECTRIC CGR S.A., 100, rue Camille-Desmoulins, F-92130 Issy les Moulineaux(FR)**

(72) Inventeur: **Carbon, Claude, THOMSON - CSF SCPI 19, avenue de Messine, F-75008 Paris(FR)**

(74) Mandataire: **Ballot, Paul Denis Jacques et al, Cabinet Ballot-Schmit 84, avenue Kléber, F-75116 Paris(FR)**

ACTORUM AG

## Description

La présente invention a pour objet un procédé de réglage d'un dispositif de radiologie. Ce depositif de radiologie est plus particulièrement du type de ceux utilisés dans le domaine médical. Il comporte des moyens pour réaliser des radioscopies et/ou des radiographies avec ou sans le secours d'amplificateurs de luminance.

Un dispositif de radiologie comporte un émetteur de rayons X, un panneau porte-patient, et un dispositif de mesure de l'irradiation produite par le tube après qu'elle ait traversé un corps à examiner placé sur le panneau. Le dispositif de mesure peut comporter une caméra de type télévision ou un film radiosensible. Le maniement des tubes à rayons X est délicat. Leur fontionnement comporte une transformation électronique-électromagnétique. Des électrons émis par une cathode viennent frapper à grande vitesse une anode. Sous l'effet des chocs, l'anode émet le rayonnement X attendu. Cependant le rapport de transformation ne vaut pas un. Une partie de l'énergie des électrons incidents se transforme en rayonnement électromagnétique basse-fréquence: en chaleur. Il en résulte que l'anode s'échauffe et que son utilisation doit être faite avec précaution.

Différentes solutions ont été imaginées pour résoudre ce problème. Dans une première solution les tubes à rayons X sont munis de dispositifs de refroidissement. Dans une autre solution l'anode des tubes et une anode tournante : elle présente au rayonnement des électrons une surface sans cesse renouvelée et qui en moyenne s'échauffe moins. Malgré ces solutions, quand la charge demandée au tube est trop forte, il est nécessaire de prévoir des suretés. Ainsi pour chaque type de tube il est mesuré un réseau de caractéristiques donnant, à puissance et à température d'anode données, la durée maximum pendant laquelle le tube peut être utilisé continûment pour que son anode atteigne une température limite. Cette température limite ne peut pas être dépassée sans qu'il en résulte pour le tube des dégradations irrémédiables. La sureté consiste à mesurer la température d'anode en continu et à couper l'alimentation du tube quand la température limite est atteinte.

De ce fontionnement en sureté a été déduit un ensemble de procédures ou méthodes d'utilisation des dispositifs de radiologie. Les méthodes, qui reviennent toutes à la même, consistent à calculer à partir de la haute tension du tube et de la dose à recevoir par un récepteur radiosensible, d'une part, et à partir de la température de départ de l'anode d'autre part, pendant combien de temps le tube va pouvoir être utilisé. Ou bien ce temps d'utilisation est inférieur au besoin et l'expérimentation est interdite parce qu'elle ne pourra pas être menée à terme; ou bien cette durée d'utilisation est supérieure au besoin et, dans la plupart des cas, il y a perte de temps. La température d'anode est une donnée inhérente au tube au moment où l'on vent l'utiliser: elle est calculée. La haute tension et la dose, ou sensibilité d'un film, sont des données d'images à réaliser. La haute tension du tube conditionne le spectre du rayonnement X c'est-à-dire le pouvoir de pénétration de ces rayons. On le détermine par expérience en fonction des zones à imager dans un corps humain. La dose, ou la sensibilité du film, est une conséquence directe de cette haute tension et des pratiques habituelles. Elle correspond au produit du courant de chauffage de la cathode du tube par la durée pendant lequel il émet. Cette durée est bien entendu la même que celle de l'utilisation du tube. Ce produit, donné en mA.s, est en définitive directement proportionnel à l'exposition du film. Il faut que le film ait reçu une dose de rayonnement suffisant pour qu'on puisse considérer qu'il a suffisamment été exposé. Le produit de la haute tension, les kilovolts Kv, par les milliampères-secondes, les mA.s, donne l'énergie radioélectrique dispensée par le tube, c'est-à-dire, au rapport de transformation près, la quantité d'échauffement qu'il en résulte pour l'anode.

Par exemple dans une méthode, un opérateur affiche séparément deux données radiographiques lés kilovolts et les milliampères-secondes. Le temps d'utilisation est calculé en tenant compte d'un abaque de charge du tube. Le défaut de cette méthode est que si on fait plusieurs poses successives, les temps d'utilisation s'allongent au fur et à mesure des expositions. En effet entre chaque exposition la température d'anode a évolué en s'échauffant : sa marge d'évolution vers la température limite diminue. Dans les générateurs sophistiqués pour parer à cet inconvénient un opérateur entre un coefficient $\underline{k}$, inférieur à 1, qui permet à un microprocesseur de faire un calcul de temps de pose avec un abaque de charge homothétique que de l'abaque de charge réel dans un facteur $\underline{k}$. Autrement dit les durées ne sont pas optimisées. Elles sont plus longues (de 1/k). Or pour l'examen des corps humains où certaines parties sont en mouvement, pour éviter un flou cinétique, il est nécessaire de choisir des temps les plus courts. En conséquence avec cette méthode si le fontionnement est sûr, il n'est pas le meilleur.

La présente invention a pour objet de remédier aux inconvénients cités en simplifiant par ailleurs le travail de l'opérateur. En effet, celui-ci n'est pas intéressé ni par la température de l'anode ni par le facteur de mérite $\underline{k}$ à attribuer à une expérimentation. Dans l'invention il suffit d'indiquer le nombre de poses N pendant lesquelles doit fonctionner le tube. Un microprocesseur calcule alors les durées d'exposition de manière à ce qu'elles soient les plus petites possible.

L'invention consiste en un procédé de réglage d'un dispositif de radiologie du type comportant les phases suivantes :
- on introduit dans un microprocesseur des données relatives à des images à réaliser avec le dispositif et relatives à un tube à rayons X à utiliser dans le dispositif, les données relatives au tube comportant notamment la valeur mesurée de la temperature initiale de l'anode du tube,
- on cancule avec ces données des valeurs de réglage du tube, et,
- on régle le tube conformément à ces valeurs, caractérisé en ce que,
- dans la phase d'introduction on indique, relativement aux images à réaliser, le nombre désiré de ces images, la haute tension de fonctionnement du tube, et la dose nécessaire à un récepteur radiosensible à utiliser pour révéler les images, et que
- dans la phase de calcul on optimise les valeurs de réglage en fonction des données d'images et des données du tube de manière à ce que les durées d'exposition soient les plus petites possible, tout en évitant une surcharge du tube.

L'invention sera mieux comprise à la lecture de la description qui suit et à l'examen des figures qui l'accompagnent. Celles-ci ne sont données qu'à titre indicatif et nullement limitatif de l'invention. Elles représentent:

- figure 1 : un dispositif pour la mise en oeuvre du procédé de l'invention;
- figure 2 : un réseau de courbes paramètrées montrant les abaques de charge du tube:
- figure 3 : un exemple d'organigramme d'opérations d'optimisation à réaliser par un microprocesseur.

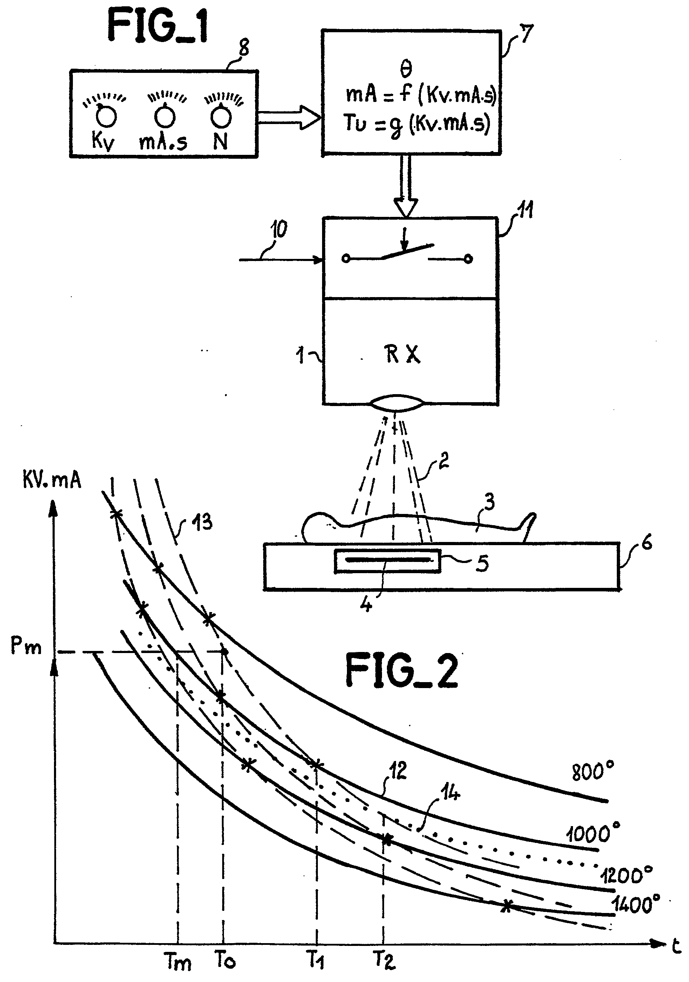

La figure 1 représente un dispositif pour la mise en oeuvre du procédé de l'invention. Il comporte un tube 1 à rayons X qui irradie 2 un corps 3. Le rayonnement qui traverse le corps 3 vient impressionner, par exemple, un film radiosensible 4 situé dans un porte-cassette 5 disposé dans un panneau porte-patient 6 à l'aplomb du corps 3. Un microprocesseur 7 reçoit des données d'image : les kilovolts déterminés expérimentalement par la nature du corps 3, les milliampères-secondes déterminés par la dose nécessaire pour impressionner le film 4 et un nombre N donnant le nombre d'images à réaliser. Quand le récepteur radiosensible est un film, le porte-cassette 5 comporte un dispositif changeur de film pour, à chaque image, présenter un autre film 4 sous le rayonnement 2. En radio-cinéma ou en application numérique, le dose nécessaire est déterminée par les caractéristiques des détecteurs utilisés. Les données d'image, appelées souvent constantes radiogiques, sont introduites au moyen d'un pupitre 8. Le microprocesseur 7 calcule par ailleurs les données relatives au tube à rayons X : essentiellement la température d'anode θ. D'une manière connue, ce calcul est prévisionnel, il tient compte de l'historique de fonctionnement du tube depuis sa mise en service. Avec ces données le microprocesseur 7 calcule le temps de pose et le courant de chauffage pour chacune des N images. Il comporte des moyens non représentés, et classiques, pour effectuer les réglages du tube. Une commande 10 permet à un opérateur de mettre en service le dispositif de radiologie une fois que les réglages sont effectués. La durée de mise en service du tube est autorisée par un interrupteur 11 qui symbolise le fonctionnement du tube pendant sa durée de travail.

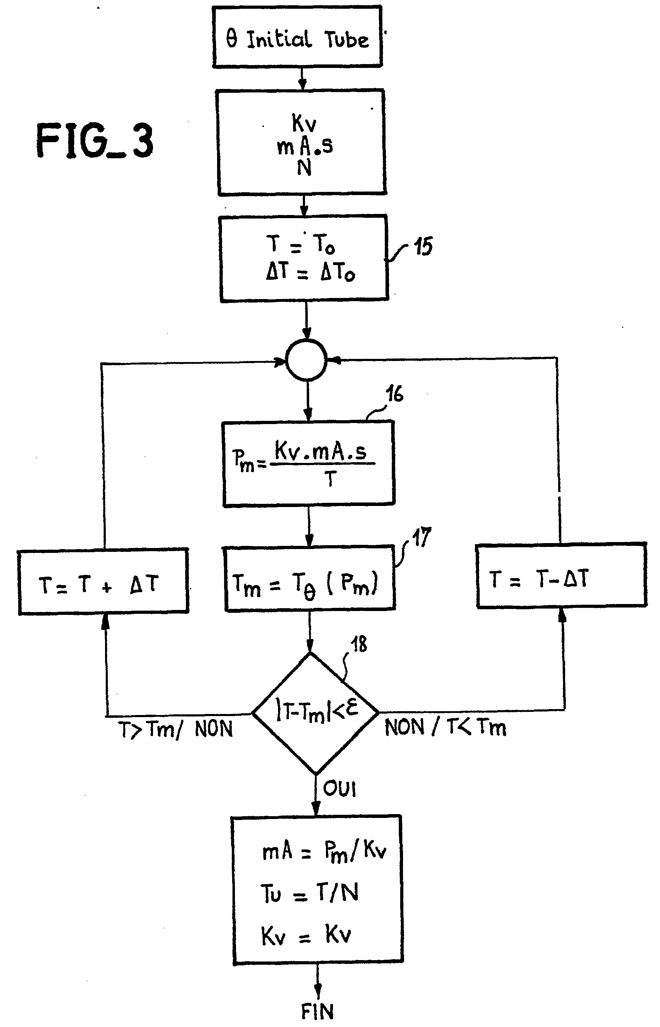

Ce qui caractérise l'invention est que, dans les paramètres introduits au moyen du pupitre 8, on indique le nombre d'images à réaliser : N. Par ailleurs le microprocesseur 7 effectue une optimisation de la durée des images pour la rendre la plus courte possible. Le fontionnement du procédé de l'invention est maintenant expliqué au regard du réseau de courbes de la figure 2 et de l'organigramme de la figure 3. Sur la figure 2 les courbes paramétrées en degrés (800° à 1400°) indiquent la durée maximum de pose, pour une puissance donnée du tube et pour une température initiale donnée de l'anode, au bout de laquelle la température d'anode atteindra la température limite. Pour une puissance donnée du tube, en ordonnée Kv.mA, et pour une température d'anode donnée, on peut déterminer, en abscisse, une durée maximum de pose envisageable. A puissance donnée, plus la température d'anode au départ de l'expérimentation est élevée, plus la durée possible d'utilisation du tube est courte. Pour une image à réaliser l'exposition ou la dose, les mA.s, sont imposées. Comme la haute tension l'est également, le produit Kv.mA.s est donné. Il en résulte des tracés hyperboliques en tirets sur le réseau de la figure 2. Chaque tracé hyperbolique correspond a une exposition donnée. Dans l'invention on calcule comme durée optimale la durée T$_1$ obtenue par l'intersection d'un abaque de charge 12 (paramétrée par la température de départ de l'anode du tube) avec une hyperbole 13 (paramétrée par une exposition à réaliser). Dans l'état de la technique décrit, la présence du facteur k conduisait en pratique à retenir un abaque de charge 14 homothétique de l'abaque 12 réel. Il en résultait le choix d'une durée T$_2$ plus longue et donc défavorable du point de vue du flou cinétique.

Dans un exemple préféré le procéé de l'invention est mis en oeuvre au moyen d'une suite d'opérations représentées schématiquement par l'organigramme de la figure 3. Au préalable le microprocesseur 7 enregistre la température θ initiale du tube, les données d'image introduites avec le pupitre 8 ainsi qu'une durée arbitraire T$_O$ et un incrément de durée arbitraire Δ T$_O$. Dans un exemple T$_O$ vaut 100 millisecondes et Δ T$_O$ vaut 10 millisecondes. Le microprocessuer comporte également dans une mémoire morte les données de tube représentées schématiquement par le réseau des abaques de charge de la figure 2. Dans un premier temps, à une phase 15, une durée d'utilisation calculée est assimilée à la durée arbitraire : T = T$_O$. Dans un deuxième temps, à une phase 16, le microprocesseur calcule une puissance de marche P$_m$ du tube. Cette puissance de marche est égale au rapport du produit Kv.mA.s divisé par le durée "calculée" T. Cette puissance de marche correspond en définitive à un point de la courbe 13. Dans une troisème phase 17 on calcule une durée de marche T$_m$. Cette durée de marche est obtenue en reportant la puissance de marche sur l'abaque de charge considéré du tube : courbe 12. Sur la figure 2, dans l'exemple, T$_m$ est inférieur à T$_O$.

Dans une quatrième phase 18 on calcule l'écart entre la durée calculée, ici T, et les durée de marche T$_m$. Si cet écart est supérieur à une précision ε donnée on modifie la durée calculée T dans un sens convenable. Dans le cas présent T étant supérieur à T$_m$, il convient d'augmenter la durée calculée T choisie. La durée T est remplacée par T + Δ T. On procède ainsi de suite par ajout (ou retrait) d'incréments de durée arbitraire Δ T jusqu'à ce que T$_m$ et T soient suffisamment voisins l'un de l'autre. Dans ce cas chacun d'eux peut être considéré comme identique à la durée T$_1$. Cette durée T$_1$ est optimum du

point de vue du flou cinétique. En effet elle est la durée la plus courte pour laquelle, si l'anode est au départ à une température θ donnée, elle se retrouve en fin de durée à la température limite. La sortie OUI de test 18 permet de régler le courant cathodique : il est égal au rapport de la dernière puissance de marche Pm à la haute tension Kv imposée pour le tube. La durée unitaire Tu de chaque image est égale à la dernière durée calculée retenue divisée par le nombre d'images qu'il y a lieu de faire. La haute tension du tube reste telle qu'imposée dès le départ. Dans ces conditions le procédé de réglage de l'invention permet d'optimiser l'utilisation des dispositifs de radiologie.

**Revendications**

1. Procédé de réglage d'un dispositif (figure 1) de radiologie du type comportant les phases suivantes:
   - on introduit (8) dans un microprocesseur (7) les données relatives à des images à réaliser (Kv, mA.s) avec le dispositif, et relatives (figure 2) à un tube à rayons X (θ) à utiliser dans le dispositif, les données relatives au tube comportant notamment la valeur mesurée de la température (q) initiale de l'anode du tube,
   - on calcule avec ces données des valeurs (kv, mA, T) de réglage du tube, et,
   - on règle (11) le tube conformément à ces valeurs, caractérisé en ce que,
   - dans la phase d'introduction on indique, relativement aux images à réaliser, le nombre (N) désiré de ces images, la haute tension (Kv) de fonctionnement du tube, est la dose (mA.s) nécessaire à un récepteur radiosensible à utiliser pour révéler les images, et que
   - dans la phase de calcul on optimise (figure 3) les valeurs de réglage en fonction des données d'images et des données (figure 2) du tube de manière à ce que les durées d'exposition soient les plus petites possible, tout en évitant une surcharge du tube.

2. Procédé selon la revendication 1, caractérisé en ce que pour optimiser on calcule les valeurs de réglage afin que les durées de prise d'image correspondent à des durées les plus courtes possible.

3. Procédé selon la revendication 2, caractérisé en ce que pour optimiser les durées, on choisit une durée arbitraire (To) et un incrément de durée également arbitraire (Δ To), on calcule (16) une puissance de marche (Pm) du tube relative à cette durée arbitraire et aux données (13) d'image introduites, on calcule (17) une durée de marche (Tm) du tube relative à cette puissance de marche et aux données (12) de tube, et on optimise, par ajout ou retrait de durées incrémentales à le durée arbitraire, la durée de marche de façon qu'elle soit peu différente de la durée incrémentée.

**Patentansprüche**

1. Verfahren zur Regelung einer Radiologievorrichtung (Figur 1) vom Typ mit den folgenden Phasen:
   - die Daten, welche sich auf Bilder (Kv, mA.s) beziehen, die mit der Vorrichtung erzeugt werden sollen und sich auf eine in der Vorrichtung anzuwendende Röntgenröhre (θ) beziehen, (Figur 2) werden in einen Mikroprozessor (7) eingegeben (8), wobei die die Röhre betreffenden Daten insbesondere den gemessenen Wert der Anfangstemperatur (θ) der Röhrenanode umfassen;
   - aus diesen Daten werden Werte (kv, mA, T) für die Regelung der Röhre berechnet, und
   - die Röhre wird gemäß diesen Werten eingestellt (11), dadurch gekennzeichnet, daß:
   - in der Einführungsphase die gewünschte Anzahl (N) der Bilder in bezug auf die zu erzeugenden Bilder, die Hochspannung (Kv) zum Betrieb der Röhre und die für einen strahlungsempfindlichen Empfänger notwendige Dosis (mA.s), die für die Entwicklung der Bilder benötigt wird, angegeben wird, und daß
   - in der Rechenphase die Einstellwerte (Fig. 3) optimiert werden in Abhängigkeit von den Bilddaten und von den Daten (Figur 2) der Röhre, so daß die Strahlenexposition so klein wie möglich gehalten werden und zugleich eine Überlastung der Röhre vermieden wird.

2. Verfahren nach Anspruch 1, dadurch gekennzeichnet, daß zur Optimierung der Bildaufnahme den kürzestmöglichen Dauern entsprechen.

3. Verfahren nach Anspruch 2, dadurch gekennzeichnet, daß für eine Optimierung der Dauer eine willkürliche Dauer (To) sowie eine ebenfalls willkürliche Inkrementierungsdauer (ΔTo) gewählt werden, daß eine Betriebsleistung (Pm) der Röhre, welche sich auf diese willkürliche Dauer sowie auf die eingeführten Bilddaten (13) bezieht, berechnet (16) wird, daß eine Betriebsdauer (Tm) der Röhre, die sich auf diese Betriebsleistung und auf die Daten (12) der Röhre beziehen, berechnet wird (17) und daß die Betriebsdauer durch Hinzufügen oder Wegnahme von Dauerinkrementen zu bzw. von der willkürlichen Dauer optimiert wird, so daß sie sich von der inkrementierten Dauer wenig unterscheidet.

**Claims**

1. A method of regulating a radiological device (figure 1) of the type comprising the following phases:
   - the input (8) to a microprocessor (7) of data relating to the images to be produced (kV, mA.s) with the device, and relating (figure 2) to an x-ray tube (9) to be utilized in the device, the data relating to the tube comprising more significantly the measured value of the initial temperature (θ) of the anode of the tube,
   - calculation, on the basis of these data, of values (kV, mA, T) for regulation of the tube,
   - regulation (11) of the tube in conformity with these values, characterized in that
   - in the phase of input there is an indication, in relation to the images to be produced, of the desired number (N) of such images, the high voltage (kV) of the operation of the tube is the dosage (mA.s) necessary for a radiosensitive receiving means to be utilized to develop the images and in that

– in the calculation phase there is an optimization (figure 3) of the regulation values as a function of the image data and the tube data (figure 2) in such a manner that the exposure data are as small as possible while nevertheless avoiding overloading the tube.

2. The method as claimed in claim 1, characterized in that in order to optimize a calculation is performed of the regulation values so that the durations of the detection of the image correspond to the shortest possible times.

3. The method as claimed in claim 2, characterized in that in order to optimize the durations an arbitrary duration ($T_0$) and a duration increment ($\Delta T_0$), which is also arbitrary, are selected, an operating power ($P_m$) for the tube is selected in relation to such arbitrary duration and to the image data (13) which are introduced, an operation duration ($T_m$) of the tube is calculated (17) in relation to this operation power and in relation to the data (12) of the tube and by the addition or subtraction of the incremental duration to or from the arbitrary duration the duration of operation is optimized in such a manner that it is a little different to the incremented duration.

FIG_1

FIG_2

EP 0 214 887 B1

## FIG_3

$\theta$ Initial Tube

Kv
mA.s
N

$T = T_o$
$\Delta T = \Delta T_o$ — 15

$P_m = \dfrac{Kv.mA.s}{T}$ — 16

$T = T + \Delta T$

$T_m = T_\theta ( P_m )$ — 17

$T = T - \Delta T$

$|T - T_m| < \varepsilon$ — 18

$T > Tm$ / NON

NON / $T < Tm$

OUI

$mA = P_m / Kv$

$Tu = T/N$

$Kv = Kv$

FIN